Europäisches Patentamt

European Patent Office

Office européen des brevets

(19)

(11) Publication number: **0 299 937**
A1

# EUROPEAN PATENT APPLICATION

(12)

(21) Application number: 88850222.6

(22) Date of filing: 21.06.88

(51) Int. Cl.⁴: **A 61 K 31/685**
A 61 K 9/50
//(A61K31/685,31:23)

(30) Priority: 06.07.87 SE 8702784

(43) Date of publication of application:
18.01.89 Bulletin 89/03

(84) Designated Contracting States:
AT BE CH DE ES FR GB GR IT LI LU NL SE

(71) Applicant: LARSSON, Kare
Norra Villavägen 7 B
S-237 00 Bjärred (SE)

Bengmark, Stig
Barytongränd 17
S-220 05 Lund (SE)

(72) Inventor: LARSSON, Kare
Norra Villavägen 7 B
S-237 00 Bjärred (SE)

Bengmark, Stig
Barytongränd 17
S-220 05 Lund (SE)

(74) Representative: Henningsson, Gunnar et al
Bergling & Sundbergh AB Box 7645
S-103 94 Stockholm (SE)

(54) A composition based on phosphatidylcholine and medical uses thereof.

(57) A new composition is disclosed which is based on phosphatidylcholine. The novel feature of said composition is that the phosphatidylcholine is present in admixture with amonoglyceride. More specifically the composition according to the invention comprises a dispersion in water or an isotonic salt solution of 0.5-20 percent by weight of a lipid mixture of 10-40 percent by weight of monoglyceride from unsaturated fatty acid having 16-22 carbon atoms and 90-60 percent by weight of phosphatidylcholine. Said monoglyceride need not be pure but may well be present in a vegetable or aninal oil.

Furthermore, said composition is disclosed for use as a prophylactic or therapeutic pharmaceutical composition as well as the use of said composition for the preparation of a pharmaceutical composition intended for the prevention of growings together of peritoneum or for the treatment of the interior of the gastro-intestinal tract.

## Description

## A composition based on phosphatidylcholine and medical uses thereof.

### TECHNICAL FIELD

The present invention relates to a phospholipid-based composition, more specifically to a composition which contains phospholipids in a new physical state. Said composition has turned out to give outstanding effects as to the prevention of growing together of peritoneum and its contents. Thus, the invention also relates to said composition for use as a prophylatic or therapeutic pharmaceutical composition as well as to the use of said composition for the preparation of a pharmaceutical composition intended to prevent the growing together of peritoneum or for the treatment of the gastro--intestinal tract.

### BACKGROUND OF THE INVENTION

In 1985 Paulo et al (Nephrologia e Dialysi, vol 2, pages 1-8) and Graham et al (Peritoneal Dialysis Bulletine, April-June 1985, pages 109-111) reported that peritoneal effluents contain phospholipids, with phosphatidylcholine as a dominating component. From their works as well as from other studies of the gastro-intestinal system and of the surface layers of the lungs it seems that beside their basic functions in all cell membranes, phospholipids also have surface-protective functions, particularly in peritoneum and in the lungs.

Thus, for quite a long time now it has been known that the pleurae are prevented from sticking together by the presence of phospholipids on the surfaces thereof. In different states of diseases this "protective layer" is damaged. It is known that in connection with certain injuries, phospholipase that is radio-actively labeled, is enriched in the organs where there are surface-active agents in abundance, e.g. in the lungs. However, it is not only phospholipase that damages these surface-active agents. Lately the interest has been very much focused on damages caused by free radicals, which are inter alia activated by radiation, light, ischemia, infection and similar.

There is a lot that indicates that the phospholipids on the surfaces of peritoneum impart to said surface a slipping action that prevents them from growing together. Therefore, it can be expected in connection with operations, peritoneal dialyses, etc., that this protective layer is partly or fully lost, which causes an increased friction followed by a growing together of said surfaces. The question which then arises is whether said protective layers can be externally replaced by the addition or application of a protective medicine.

In the light of the above-mentioned we have tested different substances as to their abilities of enhancing "the protection" and primarily to prevent the above-mentioned growing together. In initial experiments on rat considerable success has been gained with the novel composition according to the invention. In addition thereto the obtained results also make one expect the composition to work also as a gastro-intestinal pharmaceutical composition, for instance to heal ulcers in the gastro-intestinal tracts, etc,

The experiments referred to will be described more in detail below, but already at this stage it can be mentioned that our experiments show that when using phosphatidylcholine alone a radip resorption was obtained and the doses had to be repeated several times. However, when using the phosphatidylcholine in the state as is claimed by our novel composition the above-mentioned is overcome through the fact that an effect with longer duration, and probably a fusion with exactly those surfaces we intend to treat, is accomplished.

As was mentioned above the novel principle in accordan ce with the invention should also be applicable to other fields especially as concerns diseases in the gastro-intestinal tracts, e.g. ulcers, and when influencing the lungs, secondary to other diseases. In the case of an acute inflammation of pancreas - pancreatitis - the pulmonary alveoli are probably influenced by phospholipase from the pancreas. It has recently been shown that the phospholipide content of the lungs is reduced in connection with pancreatites (Guice et al, Am.J.Surg. vol 153, pages 54-61, January 1987).

As concerns ulcers or wounds inside the gastro-intestinal tract the research for the last thirty years has essentially aimed at substances causing ulcers. On the contrary there has been less interest in the prevention of ulcers. It is well known that fat-dissolving substances, such as acethyl salicyclic acid, ethyl alcohol and gallic salts, reduce the ability of the gastric mucosa of retaining hydrogen ions and protecting itself against sodium. Said substances are also known to cause the common national disease gastric catarrh or gastritis. In 1983 Butler et al (Am.J.Physiol, 1983, vol 244, pages 645-651) showed by means of thin layer cromatography the presence of a group of surface-active phospholipids similar to those surface-active agents which are present in the lungs. By the same research group said surface-active agents have been shown to be absorbed to biological membranes (Hills et al, Am.J.Physiol, 1983, vol 244, pages 561-568). Lichtenberger et al (the same group: Sience 1983, vol 219, pages 1327-2) showed that the gastric mucosa in vitro has a coating between the tissues and the contents thereof, which gives a protection against irritation. Substances like gallic salts reduce or eliminate this protection.

Swarm et al (Am. J. Surg. 1987, vol 153, pages 48-53) showed by means of experiments on dog that a mixture containing 135 µg/ml of dipalmitoyl phosphatidylcholine and 15 µg/ml of each of phasphatidyl ethanolamine, phosphatidyl glycerol, phosphatidyl inositol and sfingomyeline prevents the formation of salicyclic acid-induced acute ulcers.

## SUMMARY OF THE INVENTION

Thus, the present invention relates to a phospholipid-based composition, which has a novel contents of ingredients as compared to the above-mentioned previously known compositions and which possesses a more potent effect or an effect of a longer duration than said known compositions. More specifically the composition is characterized in that it is a dispersion in water or an isotonic salt solution of 0.5-20 percent by weight of a lipid mixture of 10-40 percent by weight of monoglyceride of unsaturated fatty acid having 16-22 carbon atoms or a vegetable or animal oil wherein fatty acids having 16-22 carbon atoms are dominating and wherein at least half of said fatty acids are unsaturated ones, and 90-60 percent by weight of phosphatidylcholine. In this context it should be noted that the percentage of the lipid mixture in said water or isotonic salt solution is based on the weight of the final dispersion, while the percentages of the lipids respectively are based on the total weight of the lipid mixture, i.e. on the sum of monoglyceride and phosphatidylcholine.

Thus, the continuous phase of the dispersion according to the invention is represented by or comprises water or an isotonic salt solution or saline. In this connection the term "isotonic salt solution" is utilized in the meaning of a salt solution having an osmotic pressure similar to that of blood, which term is well known to a person skilled in the art and need not be described further. The choice of said continuous phase is made while taking into consideration the intended use, which is a pharmaceutical one as will be disclosed more below, but in this connection it can also be added that it is previously known per se that phosphatidylcholines form a lamellar liquid-crystalline phase with water, and that it is possible at high water concentrations (above approximately 80%) to obtain stable dispersions of liposomes by a stirring action. This technique is utilized also in connection with the present invention, but by modifying the liposome dispersion of phosphatidylcholine with a certain type of lipids it has turned out possible to obtain a liposome dispersion having a changed liquid crystalline structure. Contrary to the previously known liposome dispersion this new dispersion has been shown to possess a strong tendency of adhesion to biologicial surfaces.

The reason for this effect is probably related to the phase diagram of the corresponding ternary system: phosphatidylcholine/monoglyceride/water. When the lamellar liquid-crystalline phase of phosphatidylcholine is exposed to increasing amounts of a monoglyceride of an unsaturated fatty acid in an aqueous environment, a cubic phase is formed at a critical water content. This cubic phase has the same type of structure as regions of fusion between cell membranes (see Larsson, K., J. Colloid Interface Sci. 113 (1986), 299 and Larsson, K., Acta Chem. Scand. A60 (1986), 313). With the lipid mixture of phosphatidylcholine/monoglyceride we have found a pronounced increase in adhesiveness relative to peritoneum, as is evident from the animal experiments which will be reported below; we are just below this critical composition ratio. The new liposomal dispersion can also be described as having a partly fused internal bilayer structure, as is evident from examination in a polarizing microscope and X-ray diffraction (showing both cubic and lamellar phase diffractions). These lipid particles appear to have a strong adhesiveness and are rapidly taken up.

The upper limit as to the weight percentage of the lipid mixture in the water or the isotonic salt solution is primarily chosen so as to obtain a practically useful consistency of the dispersion. Often about 20% of lipid mixture or about 80% of water or salt solution may give a very viscous dispersion which is not directly useful for many applications but which can initially be prepared in this form as a concentrate. A more preferable percentage of the lipid mixture with reference to the consistency is, however, 0.5-15, and often 0.5-10, percent by weight. Furthermore, for use of the composition in peritoneum or for gastro-intestinal applications as low concentrations of the lipid mixture as 0.5-4 percent by weight, often 1-3 percent by weight, are especially suitable, not least for a practical point of view.

With reference to the phosphatidylcholine component of the composition claimed it should be noted that it can be chosen in accordance with known principles, as said component is in fact previously known per se in similar connections. However, a preferable phosphatidylcholine is phosphatidylcholine from egg or soya, since these have been used in drugs for a long time, inter alia for intravenous applications. Thus, phosphatidylcholine is expensive and difficult to purify. Therefore, at present there is in fact no real good alternative to phosphatidylcholine from egg or soya. However, as was mentioned above the invention is not limited to any of these, since the inventive idea is applicable also to other phosphatidylcholines, including synthetic ones.

Generally the other component of the dispesion according to the invention, i.e. the monoglyceride, is a monoglyceride from an unsaturated fatty acid having 16-22 carbon atoms. However, some times it is not necessary to utilize said monoglyceride in the pure state, but in stead one may well utilize a natural product containing the same, such as a vegetable or animal oil containing the desired monoglyceride. In this context said oil should be an oil wherein fatty acids having 16-22 carbon atoms are the dominating or major constituents and wherein at least half of said fatty acids are unsaturated.

According to a preferable embodiment of the composition according to the invention the monoglyceride is a monoglyceride from an unsaturated fatty acid having 18 carbon atoms or correspondingly a vegetable or animal oil containing such a fatty acid. An especially preferable monoglyceride from said group is monoolein or monolinolin or a vegetable or animal oil containing any of these.

As examples of useful oils in accordance with the invention soya oil and sunflower oil can be mentioned.

Furthermore, with reference to the two essential ingredients of the dispersion claimed, i.e. the monoglyceride and the phosphatidylcholine, it should also be noted that they need not necessarily be utilized as one single monoglyceride in combination with one single phosphatidylcholine. Also within the scope of protection claimed is the use of a mixture of monoglycerides within the previously stated definition as well as a mixture of phosphatidylcholines, respectively.

According to a preferable embodiment of the composition according to the invention the percentage of monglyceride in the lipid mixture is 20-35 percent by weight, and an especially interesting range can in many cases be 25-30 percent by weight. This means that the corresponding percentage of phosphatidylcholine is 80-65 percent by weight and 75-70 percent by weight, respectively.

In order to further enhance adhesion of the above-mentioned neutral lipid aggregates to the exposed biosurfaces it can also be helpful to add to the composition minor amounts of cationic amphiphiles. Thus, according to a preferable embodiment of the invention the composition also contains about 1-3% (w/w) of such cationic amphiphiles based on the amount of lipids, i.e. phosphatidylcholine plus monoglyceride. A cationic lipid is preferably uitlized, which is degraded into non-toxic constituents. An ideal substance which works well is a fatty alcohol-lysine ester or the hydrochloride thereof, for example lysine-hexadecanol ester (LyC$_{16}$). In the actual aqueous media where our composition is utilized, the LyC$_{16}$ is hydrolysed into lysine and the alcohol hexadecanol within a few hours. When utilizing this way of producing positive lipid particles the preparation of composition should be kept freeze-dried and kept so until being used.

The preparation of the composition according to the invention should not cause any problems to a skilled artisan. Thus, a technique similar to that which is utilized in the manufacture of liposome dispersions in general is applicable, which manufacture is well known from thousands of published articles. Further information about said manufacture is also to be found in the working examples presented below. Moreover, a preferable method can be breafly summarized as follows. The monoglyceride and the phosphatidylcholine are dissolved in any suitable solvent so as to obtain a molecular mixture thereof, e.g. ether of ethanol. After removal of the solvent the lipid mixture will be very homogeneous. Said lipid mixture is shaked with water until a homogeneous, milky dispersion is obtained. However, as was mentioned above said dispersing operation may as well be performed in a Ringer solution and under aseptic conditions.

According to another embodiment of the invention the composition or rather the water or aqueous phase thereof optionally also contains a minor amount of polyethylene glycol, which has also turned out to increase the fusion tendency referred to above. A preferable range of said polyethylene glycol is 2-10 percent by weight based on the total amount of the major lipids of the composition, i.e. phosphatidylcholine and monoglyceride. In this context it should be noted, however, that upon frequent use of a composition containing said polyethylene glycol toxic effects may arise. On the contrary, in single case use, like after surgery, the use of polyethylene glycol to increase adhesiveness may well be motivated.

As was mentioned above the composition according to the invention has been shown to be very well suited as a pharmaceutical composition, especially to prevent or inhibit a growing together or peritoneum. Such growings may for instance arise in connection with an operation or a peritoneal dialysis. In these connections the composition claimed can thus be utilized for a prophylactic or a therapeutic treatment. The composition according to the invention should generally be utilizable for the treatment of membranes, such as gastric mucosa, intestinal mucosa, lung membranes and blood-vessel endothelium. For instance it can thus be utilized for the treatment of the internal of the gastro-intestinal tract, e.g. as a protection against or a treatment of gastric catarrh or ulcers or similar.

In other words according to another aspect of the invention there is provided the above-mentioned composition for use a a prophylactic or therapeutic pharmaceutical composition, especially intended for the prevention of growing together of peritoneum or for the treatment of the interior of the gastro-intestinal tract.

Therefore, said pharmaceutical composition should preferably be adapted for topical application or gastro-intestinal resorption, i.e. it should be prepared in accordance with the methods which are common in these connections and with such common additives which do not have any negative influences on the described effects of the present invention.

Still another aspect of the invention relates to the use of the composition for the preparation of a prophylactic or therepeutical pharmaceutical composition intended to prevent or inhibit a growing together of peritoneum or for the treatment of the interior of the gastro-intestinal tract.

## EXAMPLES

The invention will now be further described by the following, non-limiting examples.

### Example 1

10 g of egg yolk phosphatidylcholine (Sigma, purity 99%) and 4 g of glycerol monooleat (Nucheck, purity 99%) are dissolved in ethyl ether which is immediately evaporated in an evaporator under nitrogen. To the lipid mixture there are added 986 g of Ringer solution, and a shaking operation (in a shaking apparatus) is performed until a homogeneous, milky dispersion is obtained. Approximately three hours of shaking are required, and the stability follows from the fact that no lipid settles.

### Example 2

25 g of a pure soy lecithin, Epicuron 200 from the Company Lucas Meyer, Hamburg, are dispersed in 970 g of distilled water by the use of an ultrasonification rod that is dipped into the solution. Then a centrifugation for 10 minutes at 1000 g is performed to remove metal particles from the ultrasonic rod. 5 g o monoglycerides from sunflower oil, a commercial product Dimodan LS from Grindstedt Products, Brabrant, Denmark, are added, and the mixture is shaken until all Dimodan LS has been distributed throughout the dispersion. A stable dispersion is obtained.

## Example 3

14 g of egg yolk phosphatidylcholine (Sigma, purity 99%) are dispersed in 980 g of Ringer solution by a shaking similar to that of Example 1. When the dispersion is homogeneous 6 g of Dimodan LS (see Example 2) are added, and the dispersion is shaken until all monoglyceride has been taken up by the dispersion. Also in this case the dispersion is stable.

## Example 4

5 g of monoolein are added to 970 g of Ringer solution. After about one hour a cubic monoolein phase has been formed, looking like a gel, with a composition of monoolein:water of 6:4. 24 g of egg yolk phosphatidylcholine are added together with 1 g of the lysine ester of hexadecanol in the form of the dichloride salt thereof. After half an hour or one hour, when the phospholipid has swollen in the water, the solution is ultrasonified until it is homogeneous. Said ultrasonification is performed by dipping a vibrating titanium rod into the solution at such a low energy that the temperature is elevated less than 5°C above the actual temperature of the solution (usually room temperature). The liposomal dispersion obtained is heated for 10 seconds at 125°C, which is the retention time for sterilization, and filled into final storage bottles in an aseptic procedure.

## Example 5

A mixture of 8 g of monoolein and 12 g of soy bean phosphatidylcholine is kept in 980 g of distilled water for one hour until swelling is complete. Then ultrasonification is performed as described in Example 4 until a homogeneous dispersion is obtained. The solution is then freeze-dried. At use the powder is reconstituted with Ringer solution (volyme ratio of 2 to 98) to a liposomal dispersion.

## ANIMAL EXPERIMENTS

Experiments were performed on rat, where in a first series common, commercially available phosphatidylcholine was studied and compared with known substances with alleged adherence-preventing properties, viz. Varidase, starch spheres in emulsion and Allopurinol.

Standardized growings together were produced by means of illuminations for 20 minutes with light of varying wave lengths, namely ordinary light (480 nm) and UV-light (365.5 and 260 nm).

After a first treatment common phosphatidylcholine gave a very weak effect as concerns protection against growing together after illuminations with 480 and 365.5 nm and no visible effect at all after an illumination with 260 nm. After repeated treatments some protection was obtained against growings together at the first-mentioned two wave lengths, which effect was better than after a treatment with Varidase, which is considered to have the best known protective effect, and considerably better than after a treatment with starch spheres in emulsion or with Allopurinol. However, the effect was considerably weaker than that obtained by the composition according to the invention, as is disclosed below, and furthermore it could be added that in a clinical situation it is not always possible and often difficult to repeat the treatment referred to.

In a second series of experiments on rat the composition according to the invention was then tested and compared at the same type of standardized growings together as was described above in connection with the common phosphatidylcholine. The composition according to the invention turned out to have a significantly better and longer lasting adherence-preventing effect than the common phosphatidylcholine.

## Claims

1. A composition, characterized in that it is a dispersion in water or an isotonic salt or saline solution of 0.5-20 percent by weight of a lipid mixture of 10-40 percent by weight of monoglyceride of unsaturated fatty acid having 16-22 carbon atoms or vegetable or animal oil wherein fatty acids having 16-22 carbon atoms are dominating and wherein at least half of said fatty acids are unsaturated, and 90-60 percent by weight of phosphatidylcholine, the percentage of the lipid mixture being based on the weight of the final dispersion and the percentages of the respective lipids being based on the total weight of monoglyceride plus phosphatidylcholine.

2. A composition according to claim 1, characterized in tha the phosphatidylcholine is phosphatidylcholine from egg or soya.

3. A composition according to claim 1 or claim 2, characterized in that the monoglyceride is a monoglyceride from an unsaturated fatty acid having 18 carbon atoms or a vegetable or animal oil containing the same.

4. A composition according to claim 3, characterized in that the monoglyceride is monoolein or monolinolein or a vegetable or animal oil containing the same.

5. A composition according to any one of the preceding claims, characterized in that the oil is soya oil or sunflower oil.

6. A composition according to any one of the preceding claims, characterized in that the percentage of lipid mixture in the dispersion is 0.5-15, preferably 0.5-10 and especially 0.5-4 percent by weight.

7. A composition according to any one of the preceding claims, characterized in that the percentage of monoglyceride in the lipid mixture is 20-35, preferably 25-30, percent by weight.

8. A composition according to any one of the preceding claims, characterized in that it additionally contains a minor adhesion-improving amount, such as 1-3 percent by weight, of a positive or cationic lipid, preferably the hexadecyl ester of lysine or its hydrochloride, said percentage being based on the total weight of monoglyceride plus phosphatidylcholine.

9. A composition according to any one of the preceding claims, characterized in that it additionally contains a minor, adhesive-improving amount of polyethyleneglycol, preferably within the range of 2-10 percent by weight.

10. A composition according to any one of claims 1-9 for use as a prophylactic or therapeutic pharmaceutical composition.

11. A composition according to claim 10, characterized in that it is a pharmaceutical composition adapted for topical application or gastro-intestinal resorption.

12. Use of a composition according to any one of claims 1-9 for the preparation of a prophylactic or therapeutic pharmaceutical composition intended for the prevention of growing together in peritoneum or for the treatment of other membranes, such as gastric mucosa, intestinal mucosa, lung membranes or blood-vessel endothelium.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | FR-A-2 455 458 (KUREHA KAGAKU KOGYO KK)<br>* Page 6, lines 19-40; page 11, lines 8-25; page 23, lines 1-39; claims 1-9 * | 1-12 | A 61 K 31/685<br>A 61 K 9/50 //<br>(A 61 K 31/685<br>A 61 K 31:23 ) |
| Y | EP-A-0 180 786 (E. PISTOLESI)<br>* Page 10, line 1 - page 11; claims 1-10 * | 1-12 | |
| Y | EP-A-0 148 303 (A. VON MIETZKO)<br>* Page 7, line 1 - page 8, line 20; claims 1-9 * | 1-12 | |
| A | GB-A-2 080 324 (KURT KAMPFFMEYER MÜHLENVEREINIGUNG KG) | 1-12 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 20-10-1988 | BRINKMANN C. |

EPO FORM 1503 03.82 (P0401)